# EUROPEAN PATENT SPECIFICATION

(11) **EP 0 773 030 B1**
(45) Date of publication and mention of the grant of the patent: **03.09.2003**
(21) Application number: 95921113.7
(22) Date of filing: 07.06.1995
(51) Int. Cl.: A61K 39/10, C12P 21/00

(54) **METHOD OF PURIFYING NECROTOXIN PRODUCED BY BORDETELLA**
REINIGUNGSVERFAHREN FÜR VON BORDETELLA PRODUZIERTEM NEKROTOXIN
PROCEDE D'EPURATION D'UNE NECROTOXINE PRODUITE PAR BORDETELLA

(30) Priority: 10.06.1994 JP 15283494
(43) Date of publication of application: 14.05.1997
(60) Divisional application: 02028045.9
(73) Proprietor: Juridical Foundation, The Chemo-Sero-Therapeutic Research Institute, Kumamoto-shi, Kumamoto 860-8568 (JP)
(72) Inventor: KAWAI, Toru, Kumamoto 862 (JP); USHIJIMA, Toshihiro, Kumamoto 860 (JP); TAKASE, Kozo, Kumamoto 869-12 (JP); FUJIKAWA, Hideo, Kumamoto 860 (JP)
(74) Representative: VOSSIUS & PARTNER
(86) International application number: PCT/JP95/01125
(87) International publication number: WO 95/034322

(56) References cited:
- JP-A- 61 076 422
- HORIGUCHI Y. ET AL.: "Simplified procedure for purification of Bordetella bronchiseptica dermonecrotic toxin" FEMS MICROBIOLOGY LETTERS, vol. 66, 1990, pages 39-44, XP002042992
- ELIAS B. ET AL.: "Clinical and Pathological Effects of the Dermonecrotic Toxin of Bordetella bronchiseptica and Pasturella multocida in Specific-Pathogen-Free Piglets" THE JAPANESE JOURNAL OF VETERINARY SCIENCE, vol. 52, 1990, pages 677-688, XP002042993
- ZANG Y.L. AND SEKURA R.D.: "Purification and Characterization of the Heat-Labile Toxin of Bordetella pertussis" INFECTION AND IMMUNITY, vol. 59, 1991, pages 3754-3759, XP002042994
- NAKAI T. ET AL.: "Purification of Dermonecrotic Toxin from a Sonic Extract of Pasturella multocida SP-72 Serotype D" INFECTION AND IMMUNITY, vol. 46, 1984, pages 429-434, XP002042995
- NORD. VET. - MED., Vol. 36, (1984), BARFOD K. et al., pages 337-345.
- ACTA. VET. SCAND., Vol. 28, (1987), BAALSRUD K.J., pages 305-311.
- VET. QUART., Vol. 9, No. 1, (1987), DE JONG M.F. et al., pages 49-59.
- VET. REC., Vol. 124, (1989), KOBISCH M. et al., pages 57-61.
- Nordisk Veterinaer Medicin. vol. 36, 1984, p. 337-345

## Description

### TECHNICAL FIELD OF THE INVENTION

The present specification relates to a medicament for prophylaxis of swine infectious diseases. More particularly, the present specification relates to an improved toxoid of dermonecrotic toxin produced by Bordetella, a toxoid mixture comprising said toxoid and a toxoid of dermonec toxin produced by Pasteurella. The present invention relates to a process for preparing said toxoid.

### BACKGROUND OF THE INVENTION

Atrophic rhinitis (hereinafter also referred to as "AR") is a swine chronic respiratory disease which has extremely potent infectivity with primary symptoms being turbinate atrophy, hypoplasia, epistaxis, twisting of the snout and induction of other respiratory diseases, and the like. This disease is known to be caused by a toxigenic Bordetella bronchiseptica (hereinafter also referred to as "Bb") and a toxigenic Pasteurella multocida (hereinafter also referred to as "Pm") which infect at the mucosa of an upper respiratory tract. Bb possesses hemagglutinin and pili as an adherence factor and thus easily attaches to the nasal mucosa while Pm does not have such an adherence factor and hence hardly colonizes by itself. Accordingly, for the establishment of Pm infection, a predisposing factor causing a lesion at the nasal mucosa is necessary, said factor being typically Bb. Pathological factors involved in the cause of lesion in the nasal mucosa are known to be a tracheal cytotoxin and a dermonecrotic toxin. In an experimental infection, a pig is infected with Bb, and several days later, followed by intranasal infection with Pm, to cause a severe AR as observed in a field farm.

Bordetella includes B. pertussis, B. parapertussis, B. bronchiseptica, B. avium, and the like, which produce a variety of biologically active substances. Bb dermonecrotic toxin (hereinafter also referred to as "BbT") is one of such substances and is produced by any Bordetella. BbT having biological activities such as a hemorrhagic necrotic activity, an activity to cause turbinate atrophy, an activity to cause hypoplasia, a lethal activity, a vasoconstrictive activity in the smooth muscle, an activity to cause interruption in a local blood circulation, an activity to cause splenoatrophy, an activity to inhibit an antibody production, and the like was found to play an important role in Bordetellosis. Furthermore, it was found that BbT also plays an important role in the formation of pneumonic focus in piglets (Roop II, R. M. et al., Infect. Immun., 55, p.217-222 (1987)).

On the other hand, a part of Pasteurella multocida or a part of atypical Pasteurella produce Pm dermonecrotic toxin (hereinafter also referred to as "PmT"). PmT having biological activities such as a hemorrhagic necrotic activity, an activity to cause turbinate atrophy, an activity to cause hypoplasia, a lethal activity, an activity to cause interruption in a local blood circulation, an activity to cause splenoatrophy, and the like has been found to play an important role in the onset of disease by the toxigenic Pm. Furthermore, it was found that PmT is closely related to the formation of pneumonic focus in pigs by the toxigenic Pm (Iwatsu, S. and Sawada, T.; Jpn. J. Vet. Sci., 50, p.1200-1206 (1988)). Although both BbT and PmT are not secreted into a culture medium but accumulate within bacterial cells, they are released out of the bacterial cells into a culture supernatant due to bacteriolysis after a long term culture. In the in vivo process, dermonecrotic toxin released out of the bacterial cells due to bacteriolysis affect the living body.

Under the above-mentioned circumstances, the role of dermonecrotic toxin in protection in the living body still remains unclear, and for elucidating the mechanism of the protection, there is a need to develop a process for a large scale preparation of dermonecrotic toxin and to develop a toxoid of dermonecrotic toxin for use in prophylaxis of the disease. However, it has been difficult to purify dermonecrotic toxin due to its properties, i.e. unstability to heat, self-agglutination easily caused as purification processes proceed, formation of a complex with bacterial cell-derived substances such as lipids, acid proteins, hydrophobic substances, etc. Furthermore, since BbT and PmT do not immunologically cross-react to each other in spite of their similarity in biological activities, it has been earnestly desired to develop a BbT-PmT toxoid mixture which is safe and effective from the viewpoint of veterinary and pig breeding industry.

A hitherto known process for partially purifying dermonecrotic toxin from a bacterial dermonecrotic toxin-containing solution, especially from a Bordetella bacterial cell extract, includes a process using a ion exchange chromatography (Kume, K. et al., Infect. Immun., 52(4), p.370-377 (1986)). However, this process disadvantageously shows poor reproducibility. A process for partially purifying dermonecrotic toxin is also known which utilizes dialysis, a sucrose-gradient ultracentrifugation and a gel filtration chromatography (Endoh, M. et al., Microbiol. Immunol., 30, (7), p.659-673 (1986)). However, this process also shows disadvantageously poor reproducibility, and in addition, does not provide a sufficient degree of purification in spite of many steps.

Another example includes a process utilizing treatment with a nucleic acid-removing agent, an anion exchange chromatography and a hydroxyapatite adsorption chromatography (Horiguchi, Y. et al., Microbiol. Pathogen., 6, p.361-368 (1989)). However, this process is also disadvantageous in that it requires a costly reagent, a nucleic acid-removing agent, and involves many steps. There is also a process utilizing a nucleic acid-removing agent, a dialysis and a cation exchange chromatography (Horiguchi, Y. et al., FEMS Microbiol. Letters, 66, p.39-44 (1990)). Although this process shows high reproducibility and can provide dermonecrotic toxin with comparatively high purity, it also disadvantageously requires a costly reagent as in the above process.

Still another example is a process using a salting-out and a dye ligand affinity chromatography (Zhang Y. L. and Sekura R. D., Infect. Immun., 59(10), p.3754-3759 (1991)). Although this process shows a high reproducibility, it disadvantageously provides an insufficient degree of purification.

Inactivated AR vaccines reported so far includes three types, i.e. an inactivated Bb plain vaccine, an inactivated Pm plain vaccine and a Bb/Pm combined inactivated vaccine, each having been studied or put into practical use.

A Bb inactivated vaccine includes one comprising killed bacteria inactivated with formalin, glutaraldehyde, etc. supplemented with aluminum gel or an oil adjuvant (Goodnow, R.A., Vet. Med. Small Anim. Clin., 72, p.1210-1212 (1977); Nakase, Y. et al., Proc. Int. Pig Vet. Soc. Congr., 8, (1976); Giles, C.J. and Smith, I.M., Vet. Bull.(London), 53, p.327-338 (1983)). It was also found that a 68 kDa protein, which is present on the outer membrane of Bb and has an adenylate cyclase activity, exhibits a prophylactic activity against atrophic rhinitis (Montaraz J.A. et al., Infect. Immun., 47,, p.744-755 (1985)). A component vaccine comprising a Bb-produced fibrous hemagglutinin as a primary component is also known (Hansen, G.R. et al.; In Atrophic rhinitis in pigs (Ed. Peterson, K.B. and Nielsen, N.C.) Communication of the European Communities, Luxembourg, p.89-97 (1983), and Ohgitani, T. et al., Vaccine, 9, p.653-658 (1991)). There is also reported that a vaccine supplemented with dermonecrotic toxin for providing an antitoxin is not efficacious (Soderlind, O. and Bergstrom, G., Proc. Int. Pig Vet. Sci. Congr., p.175 (1984); Marel, C.M. von der. et al., Proc. Int. Pig Vet. Sci. Congr., p.170 (1984)).

On the other hand, an inactivated Pm plain vaccine includes Pm toxoid (Pedersen, K.B. and Barfod, K., Nord. Vet. Med., 31, p.293-302 (1982); Foged, N.T. et al., Vet. Rec., 125, p.7-11 (1989)), which has been studied or put into practical use.

A Bb/Pm combined inactivated vaccine includes (1) a mixture of killed Bb with killed PmT-producing PmD type bacteria, the mixture which is further supplemented with PmT non-producing bacteria, or a mixture of Bb and PmT toxoid (Barfod, K. and Pedersen, K.B., Nord. Vet. Med., 36, p.337-345 (1984); Baalsrud, K.J., Acta Vet. Scand., 28, p.305-311 (1987)); de Jong, M.F. et al., Vet. Quart., 9, p.49-59 (1987); Kobisch, M. and Pennings, A., Vet. Rec., 124, p.57-61 (1989)), for the purpose of prophylaxis of AR alone; (2) a mixture of killed Bb with killed PmD type PmT-producing bacteria and killed PmA type bacteria (Ostle, A.G. et al., Vet. Med., p.772-775 (1986)), for the purpose of prophylaxis of AR and pneumonia; and (3) a mixture of killed Bb with killed PmA type bacteria, killed Erysipelothrix insidiosa and PmT toxoid (Jayappa, H. et al., Int. Pig Vet. Soc. Congr., p.44 (1988)), for the purpose of prophylaxis of other diseases as well as AR and pneumonia, any of (1) to (3) having been put into practical use.

Since BbT plays an important role in the turbinate atrophy and the pneumonic focus formation by Bb infection, Zoop II, R.M. et al. has pointed out a need of BbT toxoid (Infect. Immun., 55, p.217-222 (1987)). However, they never refer to a combination of BbT and PmT toxoids. Chanter, N. et al. has proposed that a combination of PmT toxoid with the important toxin of Bb is more effective since the lesion in the swine nasal mucosa induced by cooperative activity of BbT and PmT provides environments favorable to Bb and Pm propagation (Res. Vet. Sci., 47, p. 48-53 (1989)).

However, a production of a BbT neutralizing antibody has not yet been successfully enhanced with a vaccine comprising a fraction having a BbT activity (Soderlind, O. and Bergstrom, G., Proc. Int. Pig Vet. Sci. Congr., p. 175 (1984); Marel, C.M. von der. et al., Proc. Int. Pig Vet. *Sci*. *Congr.,* p.170 (1984)). On the other hand, Nakai et al. revealed that a production of a neutralizing antibody is enhanced in pigs and mice with a highly purified BbT toxoid (The 111th Japan Veterinary Society, lecture abstract, p.183 (1991)). However, they also never refer to a combination of said BbT toxoid and PmT toxoid.

### DISCLOSURE OF THE INVENTION

The present inventors have earnestly studied in order to find out a method for efficiently purifying dermonecrotic toxin produced by bacteria of the species Bordetella bronchiseptica, and as a result, have found (1) that a partially purified dermonecrotic toxin with a lowered content of endotoxin can be easily obtained at a comparatively high rate of recovery by conducting a chromatography on a dermonecrotic toxin-containing solution which consists of the steps:
(1) equilibrating the chromatographic gel sulphated by direct sulfation or the chromatographic gel to which a sulphated molecule is covalently bonded or a chromatographic gel comprised of a natural or synthetic high molecular weight polymer to wich a sulfopropyl group is bonded by previously treating the gel with a buffer having pH 7 to 9 and a fixed specific electric conductivity ranging from 3 to 20 mS/cm,
(2) bringing the Bordetella bronchiseptica dermonecrotic toxin-containing solution into contact with the adsorption gel having a sulfate group as a ligand or the adsorption gel sulphated by direct sulfation under conditions of pH 7 to 9, a temperature of 0 to 30°C, and a fixed specific electric conductivity ranging from 3 to 20 mS/cm,
(3) washing the adsorption gel with a buffer having pH 5 to 10 and a fixed specific electric conductivity ranging from 3 to 20 mS/cm prior to elution of dermonecrotic toxin from the gel, and
(4) eluting dermonecrotic toxin from the gel by using a buffer having pH 5 to 10 and a specific electric conductivity of more than a fixed range of 3 to 20 mS/cm up to 130 mS/cm.

A toxoid, which is not part of the present invention is also described therein.

### BRIEF DESCRIPTION OF THE DRAWINGS

Fig. 1 shows a chromatogram in one step of a method for purifying dermonecrotic toxin according to the present invention.
Fig. 2 shows a chromatogram in one step of a method for purifying dermonecrotic toxin according to the present invention.
Fig. 3 shows a chromatogram in one step of a method for purifying dermonecrotic toxin according to the present invention.
Fig. 4 shows results of analysis of a dermonecrotic toxin fraction obtained by a method for purifying dermonecrotic toxin according to the present invention.
Fig. 5 shows results of a potency test in mice of a toxoid obtained according to the present invention.
Fig. 6 shows results of an efficacy test in pregnant sow of a toxoid obtained according to the present invention.
Fig. 7 shows results of study on protection line of a BbT neutralizing antibody.

### BEST MODE FOR CARRYING OUT THE INVENTION

The present invention provides a method for partially purifying dermonecrotic toxin which comprises bringing a dermonecrotic toxin-containing solution into contact with either a chromatographic gel sulfated by direct sulfation or a chromatographic gel to which a sulfated molecule is covalently bonded or a chromatographic gel comprised of a natural or synthehic high molecular weight polymer to which a sulfopropyl group is bonded to thereby make the dermonecrotic toxin adsorbed on the gel, followed by the elution thereof from the gel under the conditions elaborated in the appended claims. The BbT toxoid derived from BbT obtained by said method and a toxoid mixture which is prepared by mixing said BbT toxoid with a toxoid derived from PmT produced by toxigenic Pm, said toxoid mixture may be use of for prophylaxis of atrophic rhinitis in pigs.

A dermonecrotic toxin-containing solution as used herein as a starting material includes a bacterial cell extract from Bordetella bronchiseptica. The starting material of the present invention also includes an extract from cells wherein BbT is expressed by means of a genetic engineering technique. A preferred extract used herein is a bacterial cell extract from Bb. Bb is cultured in a usual manner by an agar culture on Bordet-Gengou medium, Macconkey medium, and the like, or by a standing culture, a shaking culture or an aeration spinner culture in a liquid medium such as a peptone medium (Horiguchi, Y. et al., Microb. Pathogen., 6, p.361-368 (1989)), Cohen-Willer medium, Stenner-Scholte medium, and the like. Bacterial culture on agar medium are suspended in a starting buffer of a cellulose sulfate ester gel using a Conradi stick etc. Bacterial culture in liquid medium are, after recovering bacterial cells by centrifugation, suspended in a starting buffer of a cellulose sulfate ester gel.

The obtained suspension of bacterial cells can be purified by the method of Kume, K. et al. (Infect. Immun. 52(4), p.370-377 (1986)), by the method of Endoh, M. et al. (Microbial. Immunol., 30(7), p.659-673 (1986)), by the method of Horiguchi, Y. et al. (Microbial. Pathogen., 6, p.361-368 (1988) or FEMS Microbiol. Letters, 66, p.39-44 (1990)), by the method of Zhang Y.L. and Sekura R.D. (Infect. Immun., 59(10), p.3754-3759 (1991)), or by an adsorption chromatography using either a chromatographic gel sulfated by direct sulfation such as a cellulose sulfate ester gel or a chromatographic gel to which a sulfated molecule is covalently bonded. In accordance with the present invention, however, the suspension is purified by an adsorption chromatography using either a chromatographic gel sulfated by direct sulfation or a chromatographic gel to which a sulfated molecule is covalently bonded under selected conditions.

The suspension of bacterial cells is subjected to a sonication, an enzyme treatment, a pressing, a repetition of freezing-thawing, etc. to homogenize the cells, and cellular debris are removed by centrifugation or membrane filtration. Thus obtained bacterial extract is directly, or after further pretreatment for purification via the use of a nucleic acid-removing agent, a salting-out, ultracentrifugation, etc., subjected to a method of the present invention. In accordance with the method of the present invention, the pretreatment is not always necessary but instead a bacterial cell extract can directly be subjected to an adsorption chromatography using either an adsorption gel having a sulfated molecule as a ligand or an adsorption gel sulfated by direct sulfation, thereby to make the procedure quite simple and convenient.

As used herein, a chromatographic gel sulfated by direct sulfation or a chromatographic gel to which a sulfated molecule is covalently bonded are exemplified as follows:

"A chromatographic gel sulfated by direct sulfation" as used herein means a chromatographic gel (e.g. cellulose) wherein the gel itself is directly sulfated by reaction with a sulfating agent such as chlorosulfonic acid, sulfuric anhydride in the presence of an organic solvent such as pyridine via, for example, an ester bond, including typically a cellulose sulfate ester gel wherein a sulfate ester is introduced into a globular cellulose composed of a crystalline cellulose or a crystalline region and non-crystalline region cellulose. The obtained cellulose sulfate ester retains a shape of a starting material, is insoluble in an aqueous solvent, is excellent in a physical stability, and hence is suitable for use as a chromatographic gel. These starting celluloses are already commercially available, for example, as Avicel (manufactured by Asahi Kasei Kogyo K.K.), Cellulofine GC-15, GH-25, GC-100, GC-200 (manufactured by Chisso Corporation), and the like. The starting cellulose can be sulfated by the above-mentioned methods or some sulfated cellulose are commercially available as, for example, "Sulfated Cellulofine" (manufactured by Chisso Corporation).

"A chromatographic gel to which a sulfated molecule is covalently bonded" as used herein means a chromatographic gel comprised of a natural high molecular weight polymer such as a cross-linked cellulose, a cross-linked dextran or a cross-linked agarose gel or a synthetic high molecular weight polymer such as a hydrophilic vinyl polymer or styrene divinylbenzene copolymer, to which heparin, a highly sulfated glucosaminoglycan, or sulfopropyl group, a kind of a sulfated alkyl, or a blue dye having or sulfone group is covalently bonded by CNBr procedure, etc., including many commercially available products. A chromatographic gel to which heparin is bonded includes "Heparin Sepharose CL-6B" (Pharmacia), "Affigel Heparin Gel" (Bio-Rad), "Heparin Cellulofine" (Seikagaku Kogyo K.K.), and the like. A chromatographic gel to which a blue dye is bonded includes "Affigel Blue Gel" (Bio-Rad), "AF-blue Toyopearl" (Toso K.K.), "Blue Cellulofine" (Seikagaku Kogyo K.K.), and the like. A chromatographic gel to which sulfopropyl group is bonded includes "SP-Sephadex" (Pharmacia), "SP-Toyopearl 650M" (Toso K.K.), and the like.

Purification and collection of dermonecrotic toxin from a dermonecrotic toxin-containing solution, especially a Bordetella bacterial cell extract, using an adsorption gel having a sulfate group as a ligand or an adsorption gel sulfated by direct sulfation, is carried out in the following manner. Although the present invention is illustrated by the use of a cellulose sulfate ester gel as a preferable embodiment, any other gel can also be used under similar conditions.

A cellulose sulfate ester gel is previously equilibrated with a buffer having around neutral pH (pH 7 to 9) and an appropriate specific electric conductivity, i.e. that being standardized for washing and elution in a gel chromatography (, generally 3 to 20 mS/cm, more generally 5 to 20 mS/cm although it may vary to some extent depending on a kind of a gel), for example, 0.02 M phosphate buffer supplemented with 0 to 0.2 M sodium chloride, and then subjected to an adsorption procedure for dermonecrotic toxin. A series of purification procedures such as an adsorption of dermonecrotic toxin to a cellulose sulfate ester gel, washing of the gel, elution of dermonecrotic toxin, etc. can be conducted in an industrially usual manner such as a batch process or a column process. Where a batch process is employed, a cellulose sulfate ester is placed in a Bordetella bacterial cell extract and the mixture is slowly stirred at a temperature of 0 to 30°C at a pH range of 7.0 to 9.0 for about 10 to 60 minutes to thereby make dermonecrotic toxin adsorbed onto the cellulose sulfate ester. In this case, a cellulose sulfate ester is suitably concentrated or diluted prior to adsorption procedure so that a specific electric conductivity of the Bordetella bacterial cell extract falls within the above-mentioned range, generally 3 to 20 mS/cm, or less.

After adsorption is completed, a mixture of the extract and the gel is loaded on a filter and filtration with suction is conducted to separate the gel from the filtrate. To the separated gel is poured a suitable buffer having the above fixed range of a specific electric conductivity (generally around 3 to 20 mS/cm) and pH of 5 to 10, for example, 0.02 M phosphate buffer supplemented with 0.1 M sodium chloride, to wash the gel with suction. To the gel is then poured a suitable buffer having pH of 5 to 10 and a specific electric conductivity of more than the above fixed range and generally of less than 130 mS/cm (a preferable range is, for example, around 22 to 46 mS/cm), for example, a phosphate buffer supplemented with 0.2 M to 0.5 M sodium chloride, to elute the adsorbed dermonecrotic toxin.

Where a column process is employed, conditions of a starting material, a buffer for washing and a buffer for elution may be similar to those in a batch process. It is recommended that a rate of passage of the buffers is adjusted to around 10 ml/cm²/hr to 500 ml/cm²/hr.

The method for purification according to the present invention enables specific adsorption of dermonecrotic toxin in a dermonecrotic toxin-containing solution, provides as high as several ten fold to 110-fold purification degree of dermonecrotic toxin, and shows a good recovery of dermonecrotic toxin, 40% to 100%. Endotoxin, which is responsible for side effects such as fever or shock and is contained in a large amount in an extract of bacterial cell homogenate together with dermonecrotic toxin, is not adsorbed onto the cellulose sulfate ester gel and thereby reduced to 1/30 to 1/800. The obtained purified dermonecrotic toxin shows as high as 4 x 10⁵ to 3 x 10⁶ MND/mg protein of a specific activity and forms a main band in an SDS-polyacrylamide gel electrophoresis.

As mentioned above, according to the method of the present invention, a desired dermonecrotic toxin can be obtained from a starting dermonecrotic toxin-containing solution at a good yield and purity, the procedures are quite simple, and the chromatographic adsorbent used for purification can be prepared or is available at a low cost, said adsorbent being extremely excellent from economical point of view without no deterioration after being used repeatedly. Accordingly, the method of the present invention is a quite excellent method for industrial production of dermonecrotic toxin. In addition, the method of the present invention can also be used in combination with the conventional cation exchange chromatography, gel filtration chromatography, etc. to further increase the purity to thereby provide more excellent results than those obtained by the conventional methods.

Dermonecrotic toxin purified by the method of the present invention is transformed into a toxoid which is deprived of a toxin activity while retaining immunogenicity by treatment with a chemical reagent. Transformation of dermonecrotic toxin into a toxoid can be conducted in a usual manner, for example, by using formalin (Nakai, T. et al., Infec. Immun., 46, p.429-434 (1984)) or by using glutaraldehyde (Endoh, M. et al., Microbiol. Immunol., 30, p.659-673 (1986)), etc. In order to induce humoral immunity by administering said toxoid into the living body, an adjuvant is administered together. An exemplary adjuvant to be used includes aluminum gel, oil adjuvant, saponin, etc. Said toxoid shows a higher immunogenicity than that of an unpurified fraction of dermonecrotic toxin and is also safe enough, which are confirmed by the data in Examples as described blow.

The partially purified dermonecrotic toxin obtained by the method of the present invention has a high purity and is deprived of most of endotoxin, and hence, is useful for preparing a vaccine for animal. In addition, where the purity of the partially purified dermonecrotic toxin is further increased by employing the method of the present invention in combination with the conventional purification procedures, it can also be used for preparing various reagents or medicaments or even for preparing Bordetella pertussis vaccine for human.

In most cases of natural infection of Bb, a BbT neutralizing antibody does not appear but a bacteria-agglutinating antibody increases at a high rate, which has been used for serological diagnosis of this disease. According to the present inventor's survey, only two pigs (0.6 %) in one farm (2.1 %) were detected positive for a BbT neutralizing antibody among 327 pigs in 48 farms in 15 prefectures where AR occurred. The conventional Bb killed vaccines increase a bacteria-agglutinating antibody to interfere the diagnosis. However, when a purity of BbT is further increased by combining the method for purification of the present invention with the conventional purification procedures, a toxoid can be prepared which provides a neutralizing antibody but does not increase a bacteria-agglutinating antibody. A vaccine or toxoid which enables serological distinction between a vaccine-induced antibody and an infection-induced antibody is called a "marker vaccine". For example, in specific pathogen free (SPF) farms, if by any chance Bb infection occurred while the toxoid of the present invention is used for prevention of AR, infected pigs can be detected by measuring a bacteria-agglutinating antibody.

Another aspect of the present specification descrises a toxoid mixture comprising BbT toxoid prepared in accordance with the method for purification of the present invention and a toxoid of dermonecrotic toxin produced by Pasteurella.

For PmT as used herein, a starting Pm bacterial cell extract includes a bacterial cell extract from toxigenic Pm or atypical Pasteurella (Kamp, E.M.I.E. et al., Vet. Rec., 126, p.434-437 (1990)). Alternatively, a starting material of the present invention also includes an extract of cells where PmT is expressed by a genetic engineering technique. A preferred extract used herein is a bacterial cell extract from toxigenic Pm, which is cultured in a usual manner by a standing culture, a shaking culture or an aeration spinner culture in a liquid medium such as Heart infusion medium, trypticase soy broth, Luria-Bertani medium, meat infusion medium (de Jong, M.F. and Akkermans, J.P.W.M., Vet. Quart., 8, p.294-214 (1986)) or by an agar culture such as dextrose-starch medium, blood agar medium, YPC medium (Namioka, S. and Murata, M., Cornell Vet., 51, p.498-507 (1961)), and the like. Bacterial culture on an agar medium is suspended in a Tris buffer etc. using a Conradi stick etc. Bacterial culture in a liquid medium is, after recovering bacterial cells by centrifugation, suspended in a suitable buffer for the next purification step. The suspension of bacterial cells is subjected to a sonication, an enzyme treatment, a pressing, a repetition of freezing-thawing, etc. to homogenize the cells, and cellular debris are removed by centrifugation or membrane filtration.

The cell extract is purified by an ion exchange chromatography, an affinity chromatography prepared with an antibody recognizing PmT, and the like. Alternatively, a supernatant obtained after culture of toxigenic Pm in a liquid medium for 24 to 65 hours can be used as a starting material for purification or directly subjected to a process of reducing toxicity.

The toxin fraction thus prepared is subjected to a process of detoxication by treatment with formalin, glutaraldehyde, etc. After completion of the detoxication, the buffer is replaced with a phosphate-buffered saline by dialysis etc. and thereto is added an adjuvant. An exemplary of an adjuvant which may be used includes aluminum gel, oil adjuvant, saponin, and the like. A toxoid mixture can be prepared by mixing a solution of one antigen at a concentration of twice as that of plain vaccine supplemented with an adjuvant with an equivalent amount of solution of another antigen at a concentration of twice as that of plain vaccine supplemented with an adjuvant.

The toxoid mixture obtained in accordance with the present invention not only is safe enough but also elicits both antibodies neutralizing BbT and PmT to thereby enable protection of pigs from the turbinate atrophy or hypoplasia due to both toxins.

The BbT toxoid and the toxoid mixture obtained in accordance with the present invention, when intramuscularly injected to a pregnant sow twice at an interval of 2 to 6 weeks at least one week prior to farrowing, protects their progeny from infection via a maternal antibody provided through colostrum. For the subsequent delivery, only one inoculation of the toxoid of the present invention prior to farrowing is sufficient for establishment of immunity. For the case of farms of sever AR infection, the toxoid can also effectively be intramuscularly injected to piglets of not less than 7 days old twice at an interval of 2 to 6 weeks to thereby enable direct protection.

The present invention is illustrated in more detail by means of the following Preparations and Examples,

### EXAMPLE

### Preparation 1

Chlorosulfonic acid (117 g) is added dropwise to pyridine (600 ml) at a temperature not more than 0°C and the mixture is stirred. After dropwise addition, the mixture is heated up to 65 to 70°C. Thereto is added Cellulofine GC-15 (80 g; manufactured by Chisso Corporation) and the mixture is stirred for reaction at 65 to 70°C for 3 hours. After completion of the reaction, the mixture is cooled and 10% aqueous solution of sodium hydroxide is added to gradually neutralize the mixture. The gel is filtered off and washed sufficiently with 0.01 M phosphate-buffered saline to yield a cellulose sulfate ester gel.

### Preparation 2

Chlorosulfonic acid (117 g) is added dropwise to pyridine (600 ml) at a temperature not more than 0°C and the mixture is stirred. After dropwise addition, the mixture is heated up to 65 to 70°C. Thereto is added Avicel for chromatography (80 g; microcrystalline cellulose; manufactured by Asahi Kasei Kogyo K.K.) and the mixture is retained at 65 to 70°C for 4 hours while stirring. After completion of the reaction, the mixture is cooled and 10% aqueous solution of sodium hydroxide is added to neutralize the mixture. The gel is filtered off and washed sufficiently with 0.01 M phosphate-buffered saline to yield a crystalline cellulose sulfate ester gel.

### Preparation 3

Chlorosulfonic acid (82 g) is added dropwise to pyridine (500 ml) at a temperature of 0 to 5°C and the mixture is stirred. After dropwise addition, the mixture is heated up to 65 to 70°C. Thereto is added Cellulofine GC-25 (80 g; manufactured by Chisso Corporation) and the mixture is stirred for reaction at 65 to 70°C for 4 hours. After completion of the reaction, the mixture is cooled and 10% aqueous solution of sodium hydroxide is gradually added to neutralize the mixture. The gel is filtered off and washed sufficiently with 0.01 M phosphate-buffered saline (pH 7.2) to yield a cellulose sulfate ester gel.

### Example 1

### (Gradient elution from column)

A column (50 mm (i.d.) x 200 mm) was packed with a sulfate ester of Cellulofine GC-15 prepared as in Preparation 1 and equilibrated with 0.02 M phosphate buffer (pH 8.0; specific electric conductivity, around 3 ms/cm). A cell extract (200 ml) of Bb strain S611 was applied to the column. And then, the column was washed with the above buffer to wash out debris. Then, a gradient elution was carried out with a phosphate buffer (pH 8.0; specific electric conductivity, around 3 to 46 ms/cm) supplemented with 0 to 0.5 M sodium chloride to give a fraction containing dermonecrotic toxin with 9.1 mg of a total protein. A rate of recovery of dermonecrotic toxin was 39.6% and a purification degree (specific activity of purified dermonecrotic toxin fraction / specific activity of an extract of bacterial cell homogenate) was as high as 32.1.

### Example 2

### (Step-wise elution from column)

A column (252 mm (i.d.) x 210 mm) was packed with a sulfate ester of Cellulofine GC-15 prepared as in Preparation 1 and equilibrated with 0.02 M phosphate buffer (pH 8.0; specific electric conductivity, around 3 ms/cm). A cell extract of Bb strain S611 (29 to 35 g of a total protein) was applied to the column. And then, the column was washed with 0.02 M phosphate buffer (pH 7.8 to 8.0; specific electric conductivity, around 3 to 17 ms/cm) supplemented with 0 to 0.15 M sodium chloride to wash out debris. Then, the dermonecrotic toxin was eluted with a phosphate buffer (pH 7.5 to 7.7; specific electric conductivity, around 30 to 46 ms/cm) supplemented with 0.3 to 0.5 M sodium chloride to give a fraction containing dermonecrotic toxin with 0.2 to 1.1 g of a total protein. As shown in Table 1, a rate of recovery of dermonecrotic toxin was 76 to 100% and a purification degree (specific activity of purified dermonecrotic toxin fraction / specific activity of an extract of bacterial cell homogenate) was as high as 22 to 114 with endotoxin being reduced to 0.1 to 3%.

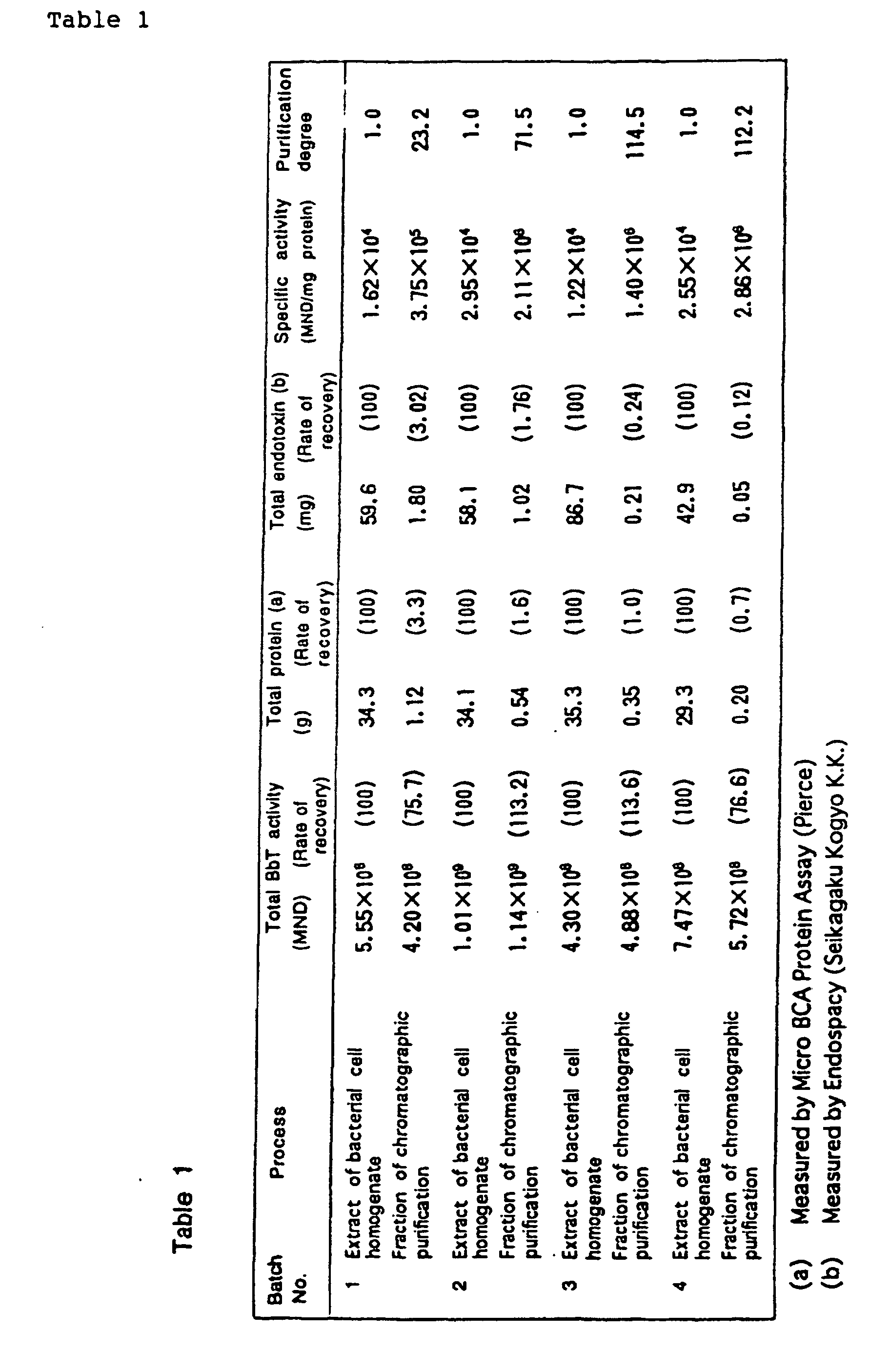

### Example 3

### (Gradient elution from Heparin Sepharose column)

A column (25 mm (i.d.) x 140 mm) was packed with Heparin Sepharose CL-6B (Pharmacia) and equilibrated with 0.02 M phosphate buffer (pH 8.0; specific electric conductivity, around 3 mS/cm). A cell extract of Bb strain S611 (420 mg of a total protein) was applied to the column. And then, the column was washed with the above buffer to wash out debris. Then, a gradient elution was carried out with a phosphate. buffer (pH 8.0; specific electric conductivity, around 3 to 46 mS/cm) supplemented with 0 to 0.5 M sodium chloride to give a fraction containing dermonecrotic toxin with 24 mg of a total protein. A rate of recovery of dermonecrotic toxin was 40.8% and a purification degree (specific activity of purified dermonecrotic toxin fraction / specific activity of an extract of bacterial cell homogenate) was as high as 7.0. The analytical results are shown in Table 2.

### Example 4

### (Gradient elution from SP-Toyopearl 650M column)

A column (50 mm (i.d.) x 140 mm) was packed with SP-Toyopearl 650M column (manufactured by Toso K.K.) and equilibrated with 0.02 M phosphate buffer (pH 8.0; specific electric conductivity, around 3 mS/cm). A cell extract of Bb strain S611 (631 mg of a total protein) was applied to the column. And then, the column was washed with the above buffer to wash out debris. Then, a gradient elution was carried out with a phosphate buffer (pH 8.0; specific electric conductivity, around 3 to 46 mS/cm) supplemented with 0 to 0.5 M sodium chloride to give a fraction containing dermonecrotic toxin with 2 mg of a total protein. A rate of recovery of dermonecrotic toxin was 11.5% and a purification degree (specific activity of purified dermonecrotic toxin fraction / specific activity of an extract of bacterial cell homogenate) was as high as 35.5. The analytical results are shown in Table 2.

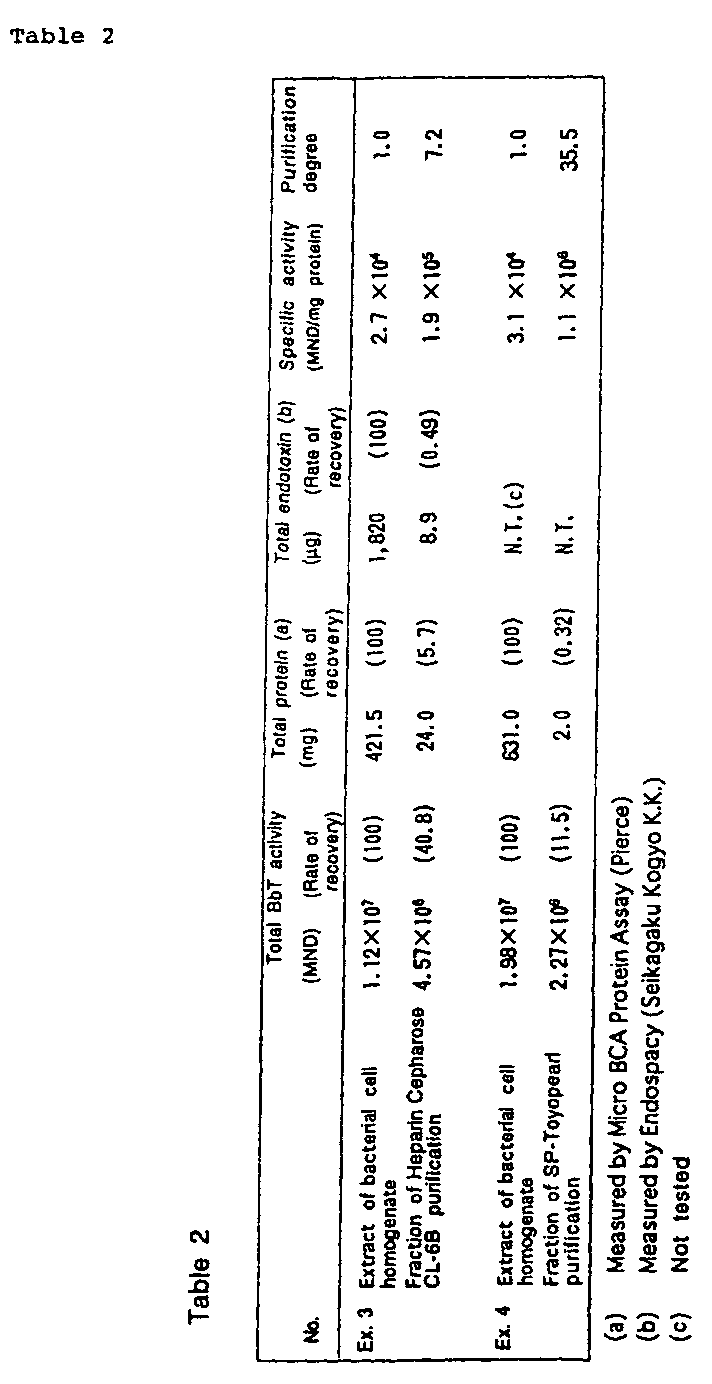

### Example 5

### (Purification with high purity)

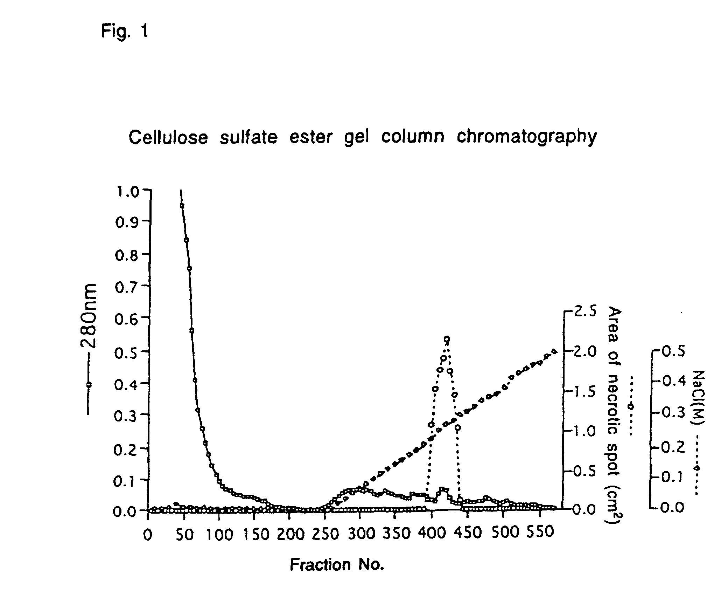

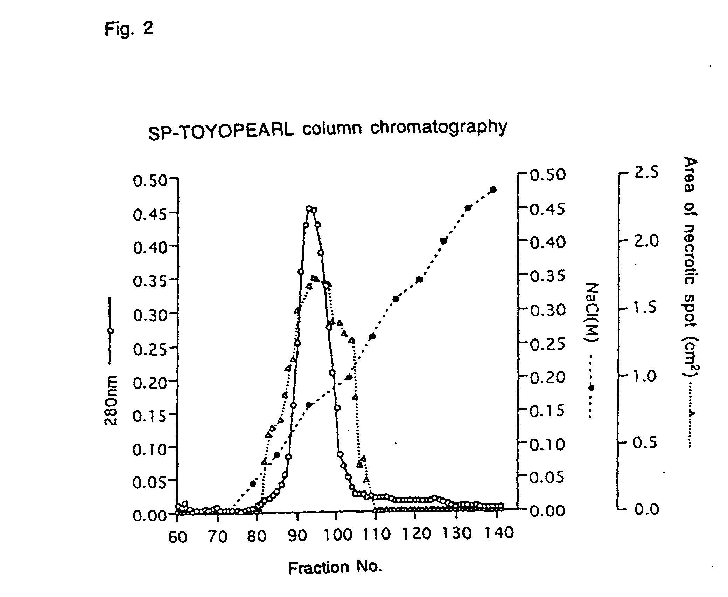

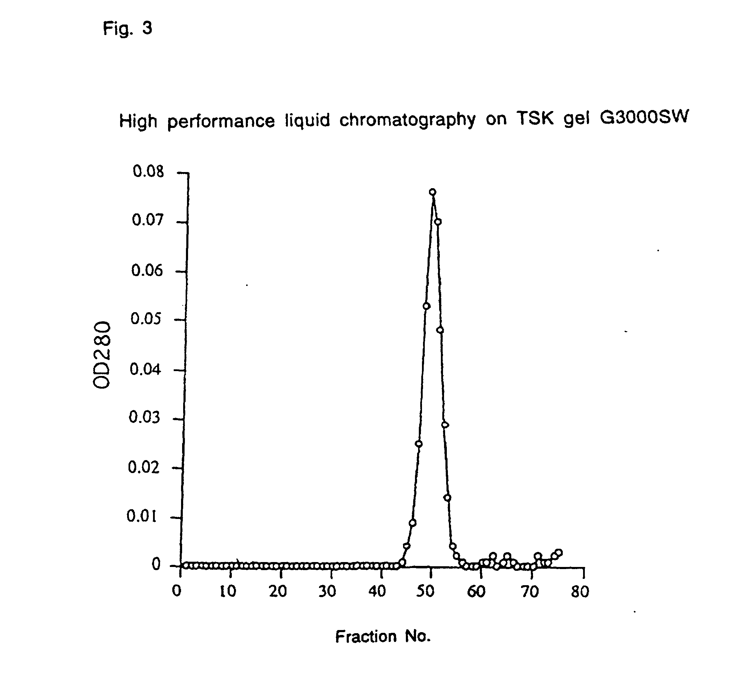

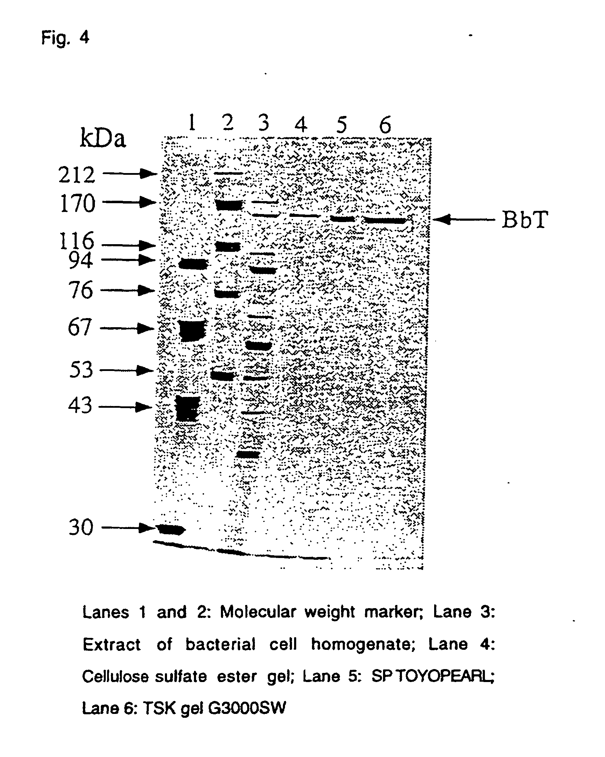

A fraction of cellulose sulfate ester gel purification prepared as in Example 1 was dialyzed against 33 mM citrate / 66 mM disodium hydrogenphosphate / 1 M urea (pH 5.0). The dialyzate (9.1 mg of a total protein) was applied to SP-Toyopearl 650M (manufactured by Toso K.K.; 10 mm (i.d.) x 80 mm) equilibrated with the same buffer, followed by washing with the same buffer to wash out debris. Then, a gradient elution was carried out with the above buffer supplemented with 0 to 0.5 M sodium chloride to give a fraction (SP fraction) containing dermonecrotic toxin (3.1 mg of a total protein). The SP fraction was further purified by a high performance liquid chromatography (HPLC) on TSK-gel G3000 SW column (21 mm (i.d.) x 600 mm) equilibrated with 50 mM phosphate buffer (pH 7.1) / 0.3 M sodium sulfate / 1M urea, to give a fraction (HPLC fraction) containing dermonecrotic toxin (1.3 mg of a total protein). These chromatograms were shown in Figs 1 to 3, respectively. The analytical results of the extract of bacterial cell homogenate and fractions of dermonecrotic toxin were shown in Table 3 and Fig. 4 and properties of the purified toxin were listed in Table 4.

**Table 3**

| Purification steps | Total BbT activity | | Total protein (a) | | Specific activity | Purification degree |
|---|---|---|---|---|---|---|
| | (MND) | (Rate of recovery) | (mg) | (Rate of recovery) | (MND/mg protein) | |
| Extract of bacterial cell homogenate | 1.7×10⁷ | (100) | 731 | (100) | 1.9×10⁴ | 1 |
| | | | | | | |
| Cellulose sulfate ester | 5.6×10⁶ | (39.6) | 9.1 | (1.2) | 6.1×10⁵ | 32 |
| | | | | | | |
| S P Toyopearl | 6.2×10⁶ | (44.3) | 3.1 | (0.4) | 2.0×10⁶ | 105 |
| | | | | | | |
| TSK G3000SW | 5.6×10⁶ | (40.0) | 1.3 | (0.2) | 4.3×10⁶ | 226 |
| | | | | | | |
| (a) According to BCA Protein Assay (manufactured by Pierce). | | | | | | |

**Table 4**

| | |
|---|---|
| Molecular weight | 146 kDa |
| Minimum dermonecrotic dose | 0.23 ng |
| Minimum amount of splenoatrophy | 0.07 ng |
| 50% Lethal dose in mice | 3.8 ng |

### Example 6

### (Reduction of toxicity)

To each dermonecrotic toxin fraction prepared as in Examples 1 to 5 was added formalin and the reaction was carried out at 37 to 40°C for 3 to 14 days for reduction of toxicity. To dermonecrotic toxin fraction was added 3/100 equivalent of 10% formalin, followed by the same amount on the next day and 2/100 equivalent on the day after the next day. After completing reduction of toxicity, the fraction was dialyzed against a phosphate-buffered saline.

### Example 7

### (Addition of adjuvant)

To the detoxified dermonecrotic toxin prepared as in Example 6 was added aluminum hydroxide gel and a preservative (thimerosal or formalin).

### Example 8

### (Potency test in mice)

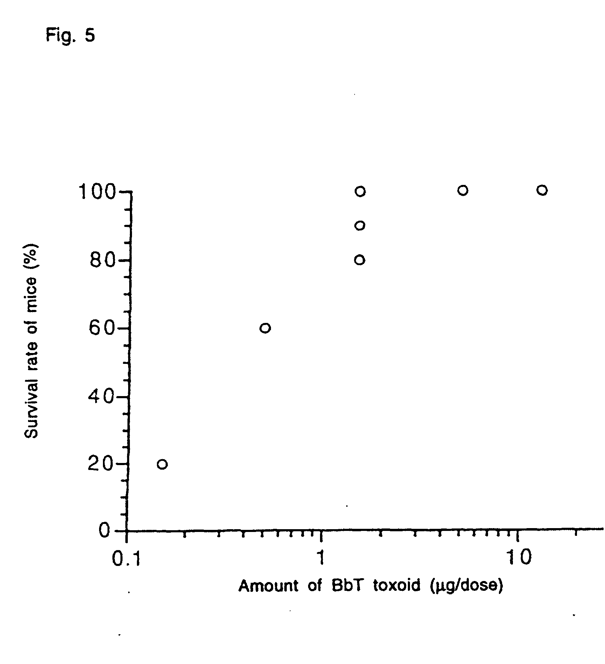

Each 0.5 ml of the toxoid obtained as in Example 7 was intraperitoneally injected to ddY mice of 5 weeks old twice at an interval of 2 weeks. Two weeks after the final immunity, the animals were challenged intraperitoneally with about 50 (25 to 100) LD₅₀ of dermonecrotic toxin and survival was observed for 7 days. The results were shown in Fig. 5. There were positive correlation between a dose of toxoid and a rate of survival in mice.

### Example 9

### (Safety test in breeding pigs)

The fraction obtained as in Example 2 was subjected to detoxication as in Example 6. The toxoid (2 to 50 µg/2 ml/dose) supplemented with aluminum hydroxide gel was prepared as in Example 7 and injected intramuscularly to SPF pigs of 10 weeks old twice at an interval of 3 weeks. No clinical symptoms or abnormality in body temperature due to injection was observed in any of tested pigs.

### Example 10

### (Efficacy test in pregnant sow)

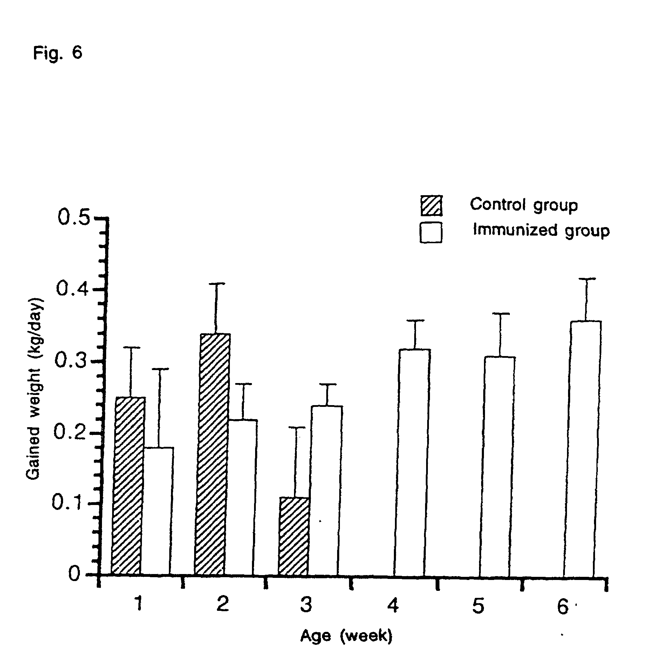

The HPLC fraction obtained as in Example 5 was subjected to reduction of toxicity as in Example 6. The toxoid (25 µg/ml) supplemented with aluminum hydroxide gel was prepared as in Example 7 and each 2 ml was injected intramuscularly to a pregnant sow twice, 6 weeks and 3 weeks prior to farrowing. Control sows were injected with a placebo vaccine. All the progenies fed on sufficient colostrum were challenged intranasally with 2 x 10⁸ viable cells of Bb strain S611 at 3 days old and the half of the progenies was further challenged intramuscularly with 2600 MND dermonecrotic toxin twice, at 2 and 3 weeks old. The obtained titer of dermonecrotic toxin neutralizing antibody was shown in Table 5. The titer of BbT neutralizing antibody was 32 in blood and 256 in colostrum of sow on farrowing, and the titer of maternal antibody in progeny was 128. The maternal antibody continued to be positive for more than 6 weeks. A sow with placebo injection and progeny thereof were seronegative. The results of the challenge test are shown in Table 6. The turbinate atrophy was detected in nine among 12 control pigs at the score of + to +++ but not in the immunized group. There was no distinction of intranasal bacterial recovery between both groups. As to a gained weight, there was difference in between both groups when challenged with dermonecrotic toxin in addition to Bb live bacteria as shown in Fig. 6. Upon the first challenge with dermonecrotic toxin at the 2 weeks old age, the gained weight ratio of the control group was reduced to about 1/3 of that of the immunized group, and as a result of the second challenge (at the 3 weeks old age), all the pigs in the control group died.

**Table 5**

| (a) at delivery | | | | | | | | | | | | | | |
|---|---|---|---|---|---|---|---|---|---|---|---|---|---|---|
| Test group | Antibody titer of sow (a) | | Titer of maternal antibody in piglets of various weeks old | | | | | | | | | | | |
| | Serum | Colostrum | 0 | | | | 2 | | | 4 | | | 6 | |
| Immunized | 32 | 256 | 128 | 128 | 128 | 64 | 64 | 32 | 16 | 32 | 32 | 16 | 16 | 32 |
| | | | 128 | 128 | | 64 | 64 | | 32 | 32 | | 16 | 16 | |
| Control | <1 | <1 | <1 | <1 | <1 | <1 | <1 | <1 | <1 | <1 | <1 | <1 | <1 | <1 |
| | | | <1 | <1 | <1 | <1 | <1 | <1 | <1 | <1 | <1 | <1 | <1 | <1 |

**Table 6**

| Group | Pig No. | Challenge with | Turbinate | Bacterial | Note |
|---|---|---|---|---|---|
| | | Bb BbT | atrophy | recovery(a) | |
| | Y126 | + - | -/-b) | ++++ | |
| | Y127 | + - | -/- | + | |
| | Y128 | + - | -/- | ++++ | |
| Immunized | Y129 | + | -/- | ++++ | |
| | Y130 | + + | -/- | + | |
| | Y131 | + + | -/- | ++++ | |
| | Y132 | + + | -/- | ++++ | |
| | Y133 | + + | -/- | + | |
| | Y134 | + + | -/- | ++++ | |
| | Y151 | + *-* | +/+ | +++ | |
| | Y152 | + - | -/+ | ++++ | |
| | Y153 | + - | -/+ | ++++ | |
| | Y154 | + - | ++/+ | ++++ | |
| | Y155 | + - | +/+ | + | |
| Control | Y156 | + - | -/- | ++++ | |
| | Y157 | + + | +++/++ | NT c) | Died at 2nd day d) |
| | Y158 | + + | +++/+++ | NT | Died at 2nd day |
| | Y159 | + + | +++/++ | NT | Died at 6th day |
| | Y160 | + + | +++/++ | NT | Died at 5th day |
| | Y161 | + + | ++/++ | NT | Died at 1st day |
| | Y162 | + + | +/+ | NT | Died at 3rd day |

| | | | | | |
|---|---|---|---|---|---|
| a) Colony number appeared on plate culture smeared with intranasal swab; : ∼0, +: ∼50, ++: ∼100, +++: ∼200, ++++: 200 - | | | | | |
| b) Assessed by five-grade (- to ++++); score of left turbinate bone/right turbinate bone | | | | | |
| c) Not tested | | | | | |
| d) Number of days counted from the second BbT challenge | | | | | |

### Example 11

### (Efficacy test in breeding pigs)

The fraction obtained as in Example 2 was subjected to reduction of toxicity as in Example 6. The toxoid (2 to 50 µg/2 ml/dose) supplemented with aluminum hydroxide gel was prepared as in Example 7 and was injected intramuscularly twice at an interval of 3 weeks to SPF pigs of 10 weeks old. Four weeks after the final immunity, the pigs were intranasally challenged with 2 x 10⁸ live Bb bacteria and a titer of dermonecrotic toxin neutralizing antibody was tested until 3 weeks after the challenge. As shown in Table 7, the control pigs remained seronegative for a BbT neutralizing antibody even after the challenge. On the other hand, all the pigs in the immunized group were converted to seropositive for a BbT neutralizing antibody. The immunized pigs injected with 2 or 6 µg/2 ml/dose of toxoid showed a radical increase in the neutralizing antibody after the challenge whereas those injected with 20 or 50 µg/2 ml/ dose did not.

It has been reported that dermonecrotic toxin has an antibody production-inhibiting activity (Sekiya, K., Microbiol. Immunol., 27, p.905-915 (1983)). The reason why the control pigs do not show increase in a neutralizing antibody after the challenge appears to be due to the immuno-suppresive activity of BbT produced by the challenging bacteria. Accordingly, since the radical increase in the neutralizing antibody in the immunized group is due to protection from the antibody production-inhibiting activity of BbT, it was suggested that 2 µg of BbT toxoid is effective in breeding pigs.

**Table 7**

| a) A dose of BbT content | b) Pig No. | Titer of BbT neutralizing antibody at various weeks after the first immunization | | | | | | Titer of Bb-agglutinating antibody at various weeks after the first immunization | | | | Intranasal bacterial recovery |
|---|---|---|---|---|---|---|---|---|---|---|---|---|
| | | 0 | 3 | 4 | 5 | 7 | 10 | 0 | 3 | 7 | 10 | |
| 0 | Y135 | <1 | <1 | <1 | <1 | <1 | <1 | <10 | <10 | <1 | 40 | ++ |
| | Y136 | <1 | <1 | <1 | <1 | <1 | <1 | <10 | <10 | <1 | 80 | ++++ |
| | | | | | | | | | | | | |
| 2 | Y137 | <1 | <1 | 16 | 8 | 8 | 128 | <10 | <10 | <10 | 640 | +++ |
| | Y138 | <1 | <1 | 16 | 32 | 16 | 512 | <10 | <10 | <10 | 1280 | +++ |
| | Y139 | <1 | <1 | 8 16 | | 16 | 1024 | <10 | <10 | <10 | 80 | ++++ |
| | | | | | | | | | | | | |
| 6 | Y140 | <1 | <1 | 16 | 32 | 16 | 512 | <10 | <10 | <10 | 640 | +++ |
| | Y141 | <1 | <1 | 16 | 64 | 16 | 128 | <10 | <1 | <10 | 320 | - |
| | Y142 | <1 | <1 | 64 | 64 | 32 | 64 | <10 | <10 | <10 | 160 | ++++ |
| | | | | | | | | | | | | |
| 20 | Y143 | <1 | <1 | 32 | 64 | 64 | 32 | <10 | <10 | <10 | 640 | ++++ |
| | Y144 | <1 | <1 | 4 | 8 | 8 | 1 | <10 | <10 | <10 | 40 | + |
| | Y145 | <1 | <1 | 16 | 32 | 8 | 8 | <10 | <1 | <10 | 320 | ++ |
| | | | | | | | | | | | | |
| 50 | Y146 | <1 | <1 | 64 | 64 | 64 | 64 | <10 | <10 | <10 | 1280 | +++ |
| | Y147 | <1 | <1 | 128 | 128 | 64 | 32 | <10 | <10 | <10 | 320 | + |
| | Y148 | <1 | <1 | 64 | 256 | 64 | 64 | <10 | <10 | <10 | 640 | ++ |
| a) µg/2 ml/dose | | | | | | | | | | | | |
| b) SPF pigs of 10 weeks old were intramuscularly injected with BbT toxoid twice at 0 and 3 weeks and challenged intranasally with live Bb at 7 week. | | | | | | | | | | | | |

### Preparation 4

### (Extract from disrupted bacterial cells: BbT toxoid 1)

Bb strain S611 was cultured in a peptone medium in fermentor with aeration at 37°C for 24 hours. The bacterial cells were concentrated by centrifugation and then mechanically disrupted. To a solution (crude BbT fraction) obtained after removing debris by centrifugation or membrane filtration (pore size: 0.45 µm) was added formalin at a final concentration of 0.8% and the toxicity was reduced at 37 to 40°C for 7 days. The obtained toxoid was dialyzed against a phosphate-buffered saline. To the dialyzed product was added aluminum hydroxide gel at 1 mg (as an amount of aluminum)/ml to give BbT toxoid 1.

### Preparation 5

### (BbT toxoid 2)

The crude BbT fraction obtained as in Preparation 4 was purified by chromatography on a cellulose sulfate ester gel column. The crude BbT was applied to a cellulose sulfate ester gel equilibrated with 20 mM phosphate buffer (pH 8.0) and the gel was washed with the same buffer. BbT was eluted from a cellulose sulfate ester gel column (7.8 cm (i.d.) x 45 cm) with 20 mM phosphate buffer (pH 8.0) supplemented with 1.5 M NaCl and 1 M urea. The eluted fraction (CS1 fraction) was subjected to the detoxication procedure as in Preparation 4, and after dialysis, aluminum hydroxide gel was added to give BbT toxoid 2.

### Preparation 6

### (BbT toxoid 3)

The CS1 fraction obtained as in Preparation 5 was further purified by a TSK gel HW65 (2.64 cm (i.d.) x 69 cm) and HW75 (2.64 cm (i.d.) x 64 cm) combination column. The CS1 fraction was passed through the column equilibrated with 0.05 M phosphate buffer (pH 7.2) supplemented with 0.3 M sodium sulfate and 1 M urea and fractionated with the same buffer, and then fractions containing the dermonecrotic toxin activity were pooled. The pooled fraction was subjected to the detoxication procedure as in Preparation 4, and after dialysis, aluminum hydroxide gel was added to give BbT toxoid 3.

### Preparation 7

### (BbT toxoid 4)

The crude BbT fraction obtained as in Preparation 4 was purified by chromatography on a cellulose sulfate ester gel column. The crude BbT was applied to a cellulose sulfate ester gel equilibrated with 20 mM phosphate buffer (pH 8.0) supplemented with 0.1 M NaCl and the gel was washed with the same buffer. BbT was eluted from a cellulose sulfate ester gel with 20 mM phosphate buffer (pH 8.0) supplemented with 0.5 M NaCl. The eluted fraction (CS2 fraction) was subjected to the detoxication procedure as in Preparation 4, and after dialysis, aluminum hydroxide gel was added to give BbT toxoid 4.

### Preparation 8

### (BbT toxoid 5)

The CS2 fraction obtained as in Preparation 7 was further purified by Affi-gel blue column (manufactured by BioRad; 100-200 mesh; 5.0 cm (i.d.) x 17 cm). The CS2 fraction dialyzed against 50 mM Tris buffer (pH 7.5) was applied to Affigel blue column equilibrated with the same buffer and the column was washed with 50 mM Tris buffer (pH 7.5) supplemented with 1 M disodium hydrogenphosphate. After the column was washed further with 50 mM Tris buffer (pH 7.5), BbT was eluted with 50 Mm Tris buffer (pH 7.5) supplemented with 2 M magnesium chloride and 1 M urea. This fraction was subjected to the detoxication procedure as in Preparation 4, and after dialysis, aluminum hydroxide gel was added to give BbT toxoid 5.

### Preparation 9

### (BbT antitoxin)

The highly purified BbT fractions obtained as in Examples 1 and 5 were subjected to the detoxication procedure as in Preparation 4, and after dialysis, aluminum hydroxide gel was added to give BbT toxoid 6. Each 1 ml of this toxoid was inoculated 8 times over 3 months to SPF pigs of 27 weeks old to give BbT antitoxin (a neutralization titer, 4,096; a titer of agglutinating antibody, <20).

### Preparation 10

### (PmT toxoid)

Toxigenic Pm type D strain of S70 were cultured in a Heart infusion broth in fermentor with aeration at 37°C for 24 hours. The bacterial cells were concentrated by centrifugation and then mechanically disrupted. A solution (abbreviated as "crude PmT") obtained after removing debris by centrifugation or membrane filtration (pore size: 0.45 µm) was purified by an anion exchange chromatography. The crude PmT was applied to the anion exchanger equilibrated with a neutral phosphate buffer supplemented with 0 to 0.3 M NaCl or Tris buffer and the exchanger was washed with the same buffer. PmT was eluted from the exchanger with the same buffer supplemented with 0.4 to 0.5 M NaCl. To this partially purified PmT fraction was added formalin at 0.4% and the toxicity was reduced at 37°C for 14 or more days. The obtained toxoid was dialyzed against a phosphate-buffered saline. To the dialyzate was added aluminum hydroxide gel at 1 mg Al/ml to give PmT toxoid.

### Example 12

### (Immunogenicity in guinea pig of BbT toxoids obtained by various purification procedures)

Each 0.5 ml of the BbT toxoids with 3,000 MND/ml (activity prior to detoxication) obtained as in Preparations 4 to 8 was intramuscularly injected to guinea pigs (Hartely; female; 5 weeks old) at the thigh. The animals were bled 28 days after the injection and the titer of a BbT neutralizing antibody was tested. Measurement of the neutralizing antibody was carried out by a two-fold serial dilution of inactivated serum with a phosphate-buffered saline. To the diluted serum was added an equal amount of BbT (titer: 4 MND/0.1 ml), and after reaction under ice-cooling overnight, each 0.1 ml of the mixture was subcutaneously injected to guinea pigs. Measurement was made one day after the injection wherein the neutralization titer was shown as a maximum dilution of serum (dilution fold of serum) which inhibited the dermonecrotic reaction.

As shown in Table 8, guinea pigs injected with BbT toxoids 1 and 2 having 1.4 x 10⁴ MND/mg protein of a specific activity remained seronegative for a neutralizing antibody. In case of guinea pigs injected with BbT toxoid 3 having 3.7 x 10⁴ MND/mg protein of a specific activity, 2 among 3 guinea pigs were seropositive, and all the animals were converted to seropositive with BbT toxoid injection of more than 7.8 x 10⁴ MND/mg protein of a specific activity.

BbT is apt to be insolubilized during purification and has properties to easily form a complex with bacterially derived materials such as lipids, acid proteins, etc. (Wordlaw, A.C. and Parton, R., Parmacol. Ther., 19, p.1-53 (1983)). Accordingly, it appears that reduction of these bacterially derived materials enables more sufficient detoxication or reduces masking of immunogenic epitope(s) on the BbT molecule so that efficiency of antigen presentation is enhanced to thereby increase the immunogenicity of BbT toxoid.

**Table 8**

| BbT Toxoid | Specific activity (MND/mg protein) | Titer of dermonecrotic toxin neutralizing Ab. | | |
|---|---|---|---|---|
| 1 | 14000 | <2 | <2 | <2 |
| 2 | 14000 | <2 | <2 | <2 |
| 3 | 37000 | <2 | 2 | 4 |
| 4 | 76000 | 32 | 32 | 128 |
| 5 | 96000 | 8 | 32 | 32 |
| Killed vaccine (Manufactured by A) | - | <2 | <2 | <2 |
| Killed vaccine (Manufactured by B) | - | <2 | <2 | <2 |

Each toxoid was adjusted to 3,000 MND/ml with addition of aluminum hydroxide gel at 1 mg Al/ml.

### Example 13

### (Protection line by BbT neutralizing antibody)

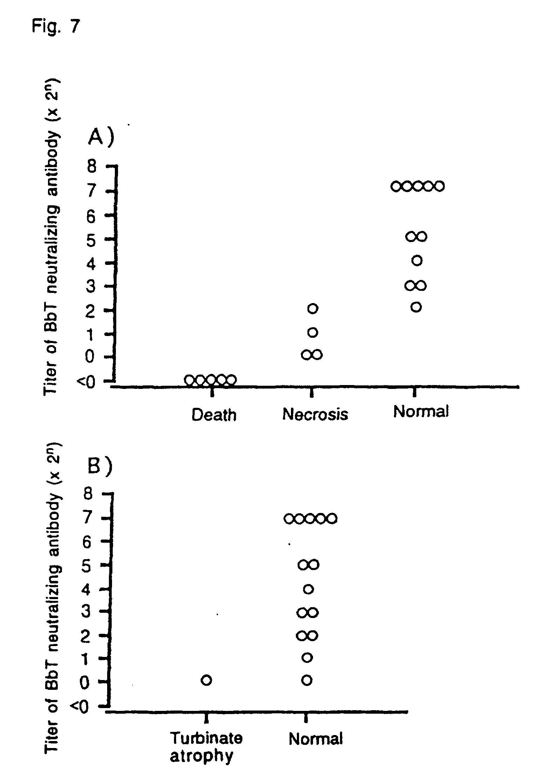

SPF pigs of 2 days old were passively immunized by intraperitoneal injection of 0.2, 1.0, 2.0 or 20 ml of BbT antitoxin obtained in Preparation 10. The pigs were intranasallly challenged at 3 days old with 10⁸ CFU of Bb strain S611 and further intramuscularly challenged at 7 days old with the crude BbT (1,940 MND/head). As shown in Fig. 7, protection was observed at a BbT neutralizing antibody titer of more than 1 against the lethal activity of BbT, whereas protection was possible with more than 4 against the dermonecrotic activity.

### Example 14

### (Protection line by PmT neutralizing antibody)

The crude PmT toxoid obtained as in Preparation 10 was emulsified with an equal amount of Freund's incomplete adjuvant. Eight SPF pregnant sows were divided into an immunization group consisting of 6 sows and a control group of 2 sows. For the immunization group, each 3 ml of toxoid (840 MND/ml) was intramuscularly injected 2 to 5 times to pigs during pregnancy. After farrowing, progeny was fed on sufficient colostrum and intramuscularly challenged at 2 or 9 days old with 20 to 160 MND of the crude PmT. The piglets were observed for 14 days after challenge and the lesion in the turbinate was checked. As shown in Table 9, protection was observed against the turbinate atrophic activity of PmT at the PmT neutralizing antibody titer of more than 2 whereas protection was possible against the lethal activity even at less than 2.

**Table 9**

| Test class | Titer of PmT neutralizing antibody a) | Death No./ Total No. b) | Score of mean turbinate atrophy c) |
|---|---|---|---|
| Immunized | 128 | 0/2 | 0 |
| | 64 | 0/2 | 0 |
| | 32 | 0/3 | 0 |
| | 16 | 0/5 | 0 |
| | 4 | 0/1 | 0 |
| | 2 | 0/9 | 0 |
| | <2 | 0/10 | 2.4 |
| Control | <2 | 3/14 | 2.3 |

| | | | |
|---|---|---|---|
| a) Titer of maternal antibody at challenge measured with embryonated bovine lung cells. | | | |
| b) Piglets of 2 or 9 days old were intramuscularly challenged at the thigh with 20 to 160 MND PmT. | | | |
| c) Mean value of score of turbinate atrophy; 0: normal, 1: slight, 2: moderate, 3: sever, 4: extremely sever | | | |

### Example 15

### (Test of Bb and Pm challenge in pigs passively immunized with BbT antitoxin)

SPF pigs of 2 days old were passively immunized by intraperitoneal injection of 20 ml of BbT antitoxin obtained in Preparation 10. The pigs were intranasally challenged at 3 and 7 days old with 10⁸ CFU of Bb strain S611 and 10⁸ CFU of Pm type D strain of S70, respectively, and necropsy was carried out at 6 weeks old. As shown in Table 10, a large quantity of Bb was recovered from the nasal mucosa when immunized with BbT antitoxin whereas recovery of Pm was much lowered in comparison with control. The turbinate lesion in the control group was assessed as sever (+++) to extreme sever (++++) whereas in the immunized group, one pig was normal (-) and the others were sever and extreme sever, showing a great variance for from individual to individual.

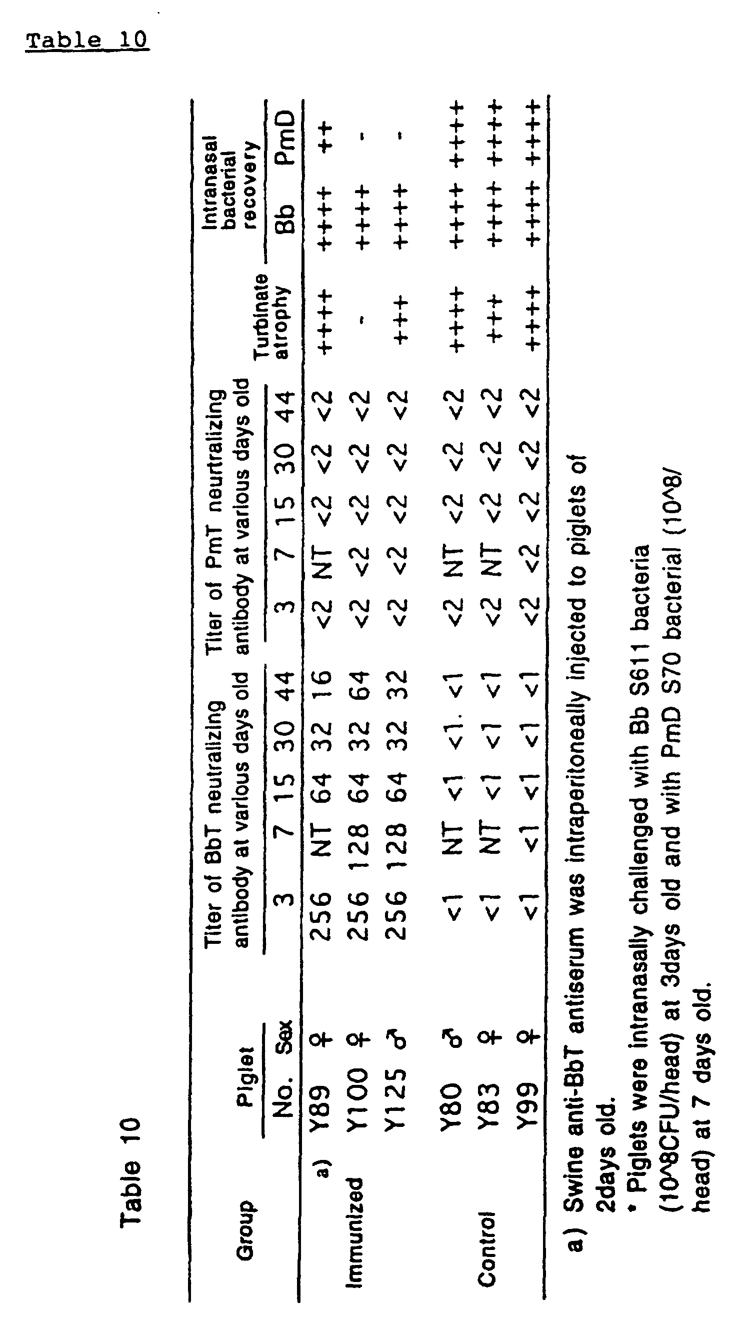

Dermonecrotic toxin is produced within the bacterial cells and secreted out of the cells by bacterial lysis. Accordingly, BbT antitoxin cannot block Bb colonization on the nasal mucosa. For Pm colonization, Bb should have previously caused the lesion at the mucosa. As a factor produced by Bb causing the lesion in the nasal mucosa, BbT (Elias, B. et al., Jpn. J. Vet. Sci., 52, p.677-688 (1990)) and a tracheal cytotoxin (Cookson, B.T. and Goldman, W.E., J. Cell. Biochem., 11B (Suppl.) p.124; Dugal, F. et al., Can. J. Vet. Res., 56, p.260-264 (1992)) are most important. Since a tracheal cytotoxin has an activity to terminate tracheal ciliary movement, an activity to destroy ciliary epithelial cells and an activity to accelerate mucus secretion, all the activities being important for colonization of Pm, immunization with BbT antitoxin can only partially block Pm colonization.

Viewing these facts, immunization against at least BbT and the tracheal cytotoxin should be established for blocking Pm colonization on the nasal mucosa. However, a tracheal cytotoxin toxoid is far from practical use, bearing many problems to be solved such as establishment of a process for purification, immunogenicity, yield, etc. Accordingly, for preventing primary symptoms of atrophic rhinitis while obviating an economical loss, an AR inactivated vaccine should comprise at least a toxoid mixture of dermonecrotic toxin.

### Example 16

### (Example of toxoid mixture)

To the detoxified BbTs obtained as in Examples 2 and 6 was added aluminum hydroxide gel and the mixture was stirred. A BbT toxoid stock solution was prepared by adjusting a concentration of BbT to 8,700 to 220,000 MND/ml (equivalent to 2 to 50 µg/ml BbT protein prior to detoxication) and a concentration of aluminum to 0.5 to 2.5 mg/ml and adding a preservative.

A PmT stock solution was prepared by preparing the detoxified PmT supplemented with aluminum hydroxide gel obtained as in Preparation 11 at 3,400 MND/ml (amount of toxin prior to detoxication) or more and adding a preservative thereto. The used preservative was 0.01% thimerosal or 0.1 to 0.4% formalin. Each equal amount of the BbT stock solution and the PmT toxoid stock solution were mixed together to give a toxoid mixture.

Each 2 ml of the toxoid mixture was intramuscularly inoculated to the neck of SPF pigs of 9 weeks old twice at an interval of 3 weeks. Serum was taken with the lapse of time and titers of a BbT neutralizing antibody and a PmT neutralizing antibody were measured. A titer of PmT neutralizing antibody was measured by a neutralization test using embryonated bovine lung cells (Rutter, J.M. and Luther, P.D., Vet. Rec., 114, p.393-396 (1984)). Table 11 shows titers of neutralizing antibodies in pigs whereas Table 12 shows results of a titer test in mice. No distinction was observed in immunogenicity between each plain toxoid of BbT and PmT and the toxoid mixture when administered to pigs and mice, and the toxoid mixture exhibited a response of neutralizing antibodies sufficient for protection and was safe enough.

**Table 11**

| Test group a) | Pig No. | Titer of BbT neutralizing antibody at various days after the first immunization | | | | | | Titer of PmT neutralizing antibody at various days after the first immunization | | | | | |
|---|---|---|---|---|---|---|---|---|---|---|---|---|---|
| | | 0 | 10 | 21 | 35 | 50 | 64 | 0 | 10 | 21 | 35 | 50 | 64 |
| BbT alone | Y117 | <1 | <1 | <1 | 32 | 32 | 8 | <2 | <2 | <2 | <2 | <2 | <2 |
| | Y118 | <1 | <1 | <1 | <1 | <1 | <1 | <2 | <2 | <2 | <2 | <2 | <2 |
| | Y119 | <1 | <1 | <1 | 64 | 16 | 8 | <2 | <2 | <2 | <2 | <2 | <2 |
| | | | | | | | | | | | | | |
| Toxoid mixture | Y120 | <1 | <1 | <1 | 64 | 32 | 16 | <2 | <2 | <2 | 256 | 128 | 128 |
| | Y121 | <1 | <1 | <1 | 16 | 8 | 4 | <2 | <2 | <2 | 64 | 32 | 16 |
| | Y122 | <1 | <1 | <1 | 2 | 2 | <1 | <2 | <2 | <2 | 64 | 32 | 32 |
| | | | | | | | | | | | | | |
| PmT alone | Y123 | <1 | <1 | <1 | <1 | <1 | <1 | <2 | <2 | <2 | 64 | 32 | 16 |
| | Y124 | <1 | <1 | <1 | <1 | <1 | <1 | <2 | <2 | <2 | 128 | 128 | 64 |
| | Y125 | <1 | <1 | <1 | <1 | <1 | <1 | <2 | <2 | <2 | 128 | 128 | 64 |
| | | | | | | | | | | | | | |
| Control | Y114 | <1 | <1 | <1 | <1 | <1 | <1 | <2 | <2 | <2 | <2 | <2 | <2 |
| | Y115 | <1 | <1 | <1 | <1 | <1 | <1 | <2 | <2 | <2 | <2 | <2 | <2 |
| | Y116 | <1 | <1 | <1 | <1 | <1 | <1 | <2 | <2 | <2 | <2 | <2 | <2 |
| a) Each toxoid was injected intramuscularly at the neck on 0 and 21 days. | | | | | | | | | | | | | |

**Table 12**

| Test group | BbT challenge | | PmT challenge | |
|---|---|---|---|---|
| | Titer of BbT neutralization | Survival No./Total | Titer of PmT No. neutralization | Survival No./Total No. |
| BbT alone | 2 5 6 | 1 0 / 1 0 | N. T. | N. T. |
| PmT alone | N. T. | N. T. | 2 5 6 | 10/10 |
| BbT.PmT mixture | 512 | 9 / 1 0 | 1 0 2 4 | 1 0 / 1 0 |
| Control | <1 | 0 / 1 0 | <2 | 0 / 1 0 |

Titer of neutralization as shown is that obtained at challenge

## Claims

1. A method for purifying dermonecrotic toxin produced by bacteria of the species Bordetella bronchiseptica which consists of the steps:
(1) equilibrating a chromatographic gel sulphated by direct sulfation or a chromatographic gel to which a sulphated molecule is covalently bonded or a chromatographic gel comprised of a natural or synthetic high molecular weight polymer to which a sulfopropyl group is bonded by previously treating the gel with a buffer having pH 7 to 9 and a fixed specific electric conductivity ranging from 3 to 20 mS/cm,
(2) bringing the Bordetella bronchiseptica dermonecrotic toxin-containing solution into contact with the adsorption gel having a sulfate group as a ligand or the adsorption gel sulphated by direct sulfation under conditions of pH 7 to 9, a temperature of 0 to 30°C, and a fixed specific electric conductivity ranging from 3 to 20 mS/cm,
(3) washing the adsorption gel with a buffer having pH 5 to 10 and a fixed specific electric conductivity ranging from 3 to 20 mS/cm prior to elution of dermonecrotic toxin from the gel, and
(4) eluting dermonecrotic toxin from the gel by using a buffer having pH 5 to 10 and a specific electric conductivity of more than a fixed range of 3 to 20 mS/cm up to 130 mS/cm.

2. The method of claim 1 which consists of the steps:
(1) equilibrating a gel of a cellulose sulfate ester by previously treating the gel with a buffer having pH 7 to 9 and a specific electric conductivity of 20 mS/cm,
(2) bringing the Bordetella bronchiseptica dermonecrotic toxin-containing solution into contact with the gel of a cellulose sulfate ester under conditions of pH 7 to 9, a temperature of 0 to 30°C, and a specific electric conductivity of 20 mS/cm,
(3) washing the adsorption gel with a buffer having pH 5 to 10 and a specific electric conductivity of 20 mS/cm prior to elution of dermonecrotic toxin from the gel, and
(4) eluting dermonecrotic toxin from the gel by using a buffer having pH 5 to 10 and a specific electric conductivity ranging from 22 to 130 mS/cm.

3. The method of claim 1 which consists of the steps:
(1) equilibrating a heparin-bound chromatographic gel by previously treating the gel with a buffer having pH 7 to 9 and a specific electric conductivity ranging from 3 to 5 mS/cm,
(2) bringing the Bordetella bronchiseptica dermonecrotic toxin-containing solution into contact with the heparin-bound chromatographic gel under conditions of pH 7 to 9, a temperature of 0 to 30°C, and a specific electric conductivity ranging from 3 to 5 mS/cm,
(3) washing the adsorption gel with a buffer having pH 5 to 10 and a specific electric conductivity ranging from 3 to 5 mS/cm or less prior to elution of dermonecrotic toxin from the gel, and
(4) eluting dermonecrotic toxin from the gel by using a buffer having pH 5 to 10 and a specific electric conductivity ranging from 3 to 130 mS/cm.

4. The method of any one of claims 1 to 3, wherein the Bordetella bronchiseptica dermonecrotic toxin-containing solution is a Bordetella bronchiseptica bacterial cell extract.

5. The method of any one of claims 1 to 3, wherein the Bordetella bronchiseptica dermonecrotic toxin-containing solution is a culture or an extract of a microorganism or a cell in which dermonecrotic toxin of Bordetella bronchiseptica is expressed by means of a genetic engineering technique.

## Patentansprüche

1. Verfahren zur Reinigung von dermonekrotischem Toxin, das von Bakterien der Gattung Bordetella bronchiseptica produziert wird, bestehend aus den Schritten:
(1) Equilibrieren eines durch direkte Sulfatierung sulfatierten Chromatographiegels oder eines Chromatographiegels, an das ein sulfatiertes Molekül kovalent gebunden ist, oder eines Chromatographiegels, bestehend aus einem natürlichen oder synthetischen Polymer mit einem hohen Molekulargewicht, an das eine Sulfopropylgruppe gebunden ist, durch vorhergehendes Behandeln des Gels mit einem Puffer mit einem pH-Wert von 7 bis 9 und einer festen, spezifischen elektrischen Leitfähigkeit im Bereich von 3 bis 20 mS/cm,
(2) Inkontaktbringen der dermonekrotisches Toxin von Bordetella bronchiseptica enthaltenden Lösung mit dem Adsorptionsgel mit einer Sulfatgruppe als Ligand oder dem durch direkte Sulfatierung sulfatierten Adsorptionsgel unter folgenden Bedingungen: ein pH-Wert von 7 bis 9, eine Temperatur von 0 bis 30°C und eine feste spezifische elektrische Leitfähigkeit im Bereich von 3 bis 20 mS/cm,
(3) Waschen des Adsorptionsgels mit einem Puffer mit einem pH-Wert von 5 bis 10 und einer festen, spezifischen elektrischen Leitfähigkeit im Bereich von 3 bis 20 mS/cm vor der Elution des dermonekrotischen Toxins vom Gel, und
(4) Eluieren des dermonekrotischen Toxins vom Gel unter Verwendung eines Puffers mit einem pH-Wert von 5 bis 10 und einer spezifischen elektrischen Leitfähigkeit über einem festen Bereich von 3 bis 20 mS/cm bis 130 mS/cm.

2. Verfahren nach Anspruch 1, bestehend aus den Schritten:
(1) Equilibrieren eines Gels aus einem Cellulosesulfatester durch vorheriges Behandeln des Gels mit einem Puffer mit einem pH-Wert von 7 bis 9 und einer spezifischen elektrischen Leitfähigkeit von 20 mS/cm,
(2) Inkontaktbringen der dermonekrotisches Toxin von Bordetella bronchiseptica enthaltenden Lösung mit dem Gel aus einem Cellulosesulfatester unter folgenden Bedingungen: ein pH-Wert von 7 bis 9, eine Temperatur von 0 bis 30°C und eine spezifische elektrische Leitfähigkeit von 20 mS/cm,
(3) Waschen des Adsorptiansgels mit einem Puffer mit einem pH-Wert von 5 bis 10 und einer spezifischen elektrischen Leitfähigkeit von 20 mS/cm vor Elution des dermonekrotischen Toxins vom Gel, und
(4) Eluieren des dermonekrotischen Toxins vom Gel unter Verwendung eines Puffers mit einem pH-Wert von 5 bis 10 und einer spezifischen elektrischen Leitfähigkeit im Bereich von 22 bis 130 mS/cm.

3. Verfahren nach Anspruch 1, bestehend aus den Schritten:
(1) Equilibrieren eines Chromatographiegels, an das Heparin gebunden ist, durch vorherige Behandlung des Geis mit einem Puffer mit einem pH-Wert von 7 bis 9 und einer spezifischen elektrischen Leitfähigkeit im Bereich von 3 bis 5 mS/cm,
(2) Inkontaktbringen der dermonekrotisches Toxin von Bordetella bronchiseptica enthaltenden Lösung mit dem Chromatographiegel, an das Heparin gebunden ist, unter folgenden Bedingungen: ein pH-Wert von 7 bis 9, eine Temperatur von 0 bis 30°C und eine spezifische elektrische Leitfähigkeit im Bereich von 3 bis 5 mS/cm,
(3) Waschen des Adsorptionsgels mit einem Puffer mit einem pH-Wert von 5 bis 10 und einer spezifischen elektrischen Leitfähigkeit im Bereich von 3 bis 5 mS/cm oder weniger vor dem Eluieren des dermonekrotischen Toxins vom Gel, und
(4) Eluieren des dermonekrotischen Toxins vom Gel unter Verwendung eines Puffers mit einem pH-Wert von 5 bis 10 und einer spezifischen elektrischen Leitfähigkeit im Bereich von 3 bis 130 mS/cm.

4. Verfahren nach einem der Ansprüche 1 bis 3, wobei die dermonekrotisches Toxin von Bordetella bronchiseptica enthaltende Lösung ein bakterieller Zellextrakt von Bordetella bronchiseptica ist.

5. Verfahren nach einem der Ansprüche 1 bis 3, wobei die dermonekrotisches Toxin von Bordetalla bronchiseptica enthaltende Lösung eine Kultur oder ein Extrakt eines Mikroorganismus oder einer Zelle ist, in der dermonekrotisches Toxin von Bordetella bronchiseptica mit Hilfe eines gentechnischen Verfahrens exprimiert ist.

## Revendications

1. Méthode de purification de la toxine dermonécrotique produite par des bactéries de l'espèce *Bordetella bronchiseptica* qui consiste en les étapes :
(1) d'équilibrage d'un gel de chromatographie sulfaté par sulfatation directe ou d'un gel de chromatographie auquel une molécule sulfatée est liée de façon covalente ou d'un gel de chromatographie comprenant un polymère naturel ou synthétique de haut poids moléculaire auquel un groupe sulfopropyle est lié, par traitement préalable du gel avec un tampon ayant un pH compris entre 7 et 9 et une conductivité électrique spécifique fixée comprise entre 3 et 20 mS/cm,
(2) de mise en contact de la solution contenant la toxine dermonécrotique de *Bordetella bronchiseptica* avec le gel d'adsorption ayant un groupe sulfate en tant que ligand ou le gel d'adsorption sulfaté par sulfatation directe, dans des conditions de pH compris entre 7 et 9, une température comprise entre 0 et 30°C, et une conductivité électrique spécifique fixée comprise entre 3 et 20 mS/cm,
(3) de lavage du gel d'adsorption avec un tampon ayant un pH compris entre 5 et 10 et une conductivité électrique spécifique fixée comprise entre 3 et 20 mS/cm avant l'élution de la toxine dermonécrotique du gel, et
(4) d'élution de la toxine dermonécrotique du gel grâce à l'utilisation d'un tampon ayant un pH compris entre 5 et 10 et une conductivité électrique spécifique supérieure à un intervalle fixé de 3 à 20 mS/cm jusqu'à 130 mS/cm.

2. Méthode selon la revendication 1 qui consiste en les étapes :
(1) d'équilibrage d'un gel à base de surate ester de cellulose par traitement préalable du gel avec un tampon ayant un pH compris entre 7 et 9 et une conductivité électrique spécifique de 20 mS/cm,
(2) de mise en contact de la solution contenant la toxine dermonécrotique de *Bordetella bronchiseptica* avec le gel à base de sulfate ester de cellulose dans des conditions de pH compris entre 7 et 9, une température comprise entre 0 et 30°C, et une conductivité électrique spécifique de 20 mS/cm,
(3) de lavage du gel d'adsorption avec un tampon ayant un pH compris entre 5 et 10 et une conductivité électrique spécifique de 20 mS/cm avant l'éiution de la toxine dermonécrotique du gel, et
(4) d'élution de la toxine dermonécrotique du gel grâce à l'utilisation d'un tampon ayant un pH compris entre 5 et10 et une conductivité électrique spécifique comprise entre 22 et 130 mS/cm.

3. Méthode selon la revendication 1 qui consiste en les étapes :
(1) d'équilibrage d'un gel de chromatographie lié à une héparine par traitement préalable du gel avec un tampon ayant un pH compris entre 7 et 9 et une conductivité électrique spécifique comprise entre 3 et 5 mS/cm.
(2) de mise en contact de la solution contenant la toxine dermonécrotique de *Bordetella bronchiseptica* avec le gel de chromatographie lié à une héparine dans des conditions de pH compris entre 7 et 9, une température comprise entre 0 et 30°C, et une conductivité électrique spécifique comprise entre 3 et 5 mS/cm,
(3) de lavage du gel d'adsorption avec un tampon ayant un pH compris entre 5 et 10 et une conductivité électrique spécifique comprise entre 3 et 5 mS/cm ou inférieure, avant l'élution de la toxine dermonécrotique du gel, et
(4) d'élution de la toxine dermonécrotique du gel grâce à l'utilisation d'un tampon ayant un pH compris entre 5 et10 et une conductivité électrique spécifique comprise entre 3 et 130 mS/cm.

4. Méthode selon l'une quelconque des revendications 1 à 3. **caractérisée en ce que** la solution contenant la toxine dermonécrotique de *Bordetella* *bronchiseptica* est un extrait cellulaire bactérien de *Bordetella bronchiseptica*.

5. Méthode selon l'une quelconque des revendications 1 à 3, **caractérisée en ce que** la solution contenant la toxine dermonécrotique de *Bordetella bronchiseptica* est une culture ou un extrait d'un microorganisme ou d'une cellule dans laquelle la toxine dermonécrotique de *Bordetella bronchiseptica* est exprimée aux moyens des techniques de génie génétique.
